# FASCICULE DE BREVET EUROPEEN

(11) **EP 1 327 435 B1**
(45) Date de publication et mention de la délivrance du brevet: **01.08.2007**
(21) Numéro de dépôt: 02293054.9
(22) Date de dépôt: 10.12.2002
(51) Int. Cl.: A61K 8/34, A61K 8/73, A61Q 1/10

(54) **Fard a paupieres comprenant une gomme de xanthane et un silicate mixte**
Lidschatten mit einem Xanthangummi und einem gemischten Silikat
Eye shadow with a xanthan gum and a mixed silicate

(30) Priorité: 09.01.2002 FR 0200214
(43) Date de publication de la demande: 16.07.2003
(73) Titulaire: L'ORÉAL, 75008 Paris (FR)
(72) Inventeur: Collin, Nathalie, 92330 Sceaux (FR); Bichon, Yohann, 75013 Paris (FR); Pays, Karl, 94550 Chevilly-Larue (FR)
(74) Mandataire: Boulard, Denis

(56) Documents cités:
- US-A- 4 604 281
- US-A- 4 994 264
- US-A- 5 840 285
- DATABASE CHEMICAL ABSTRACTS [en ligne] retrieved from STN Database accession no. 98: 40 421 XP002222975 & JP 57 171907 A (KANEBO, LTD) 22 octobre 1982 (1982-10-22)

## Description

La présente invention a pour objet une composition cosmétique de fard-à-paupières comprenant de la gomme de xanthane et un silicate mixte. L'invention a aussi pour objet un procédé de maquillage des paupières utilisant la composition.

Les fards à paupières sont constituées d'un véhicule approprié et de différentes matières colorantes destinées à conférer une certaine couleur au fard et après leur application sur les paupières. Ils peuvent se présenter sous forme aqueuse ou anhydre.

L'inconvénient rencontré le plus fréquemment lors de l'utilisation des fards à paupières réside dans le fait qu'au bout d'un certain temps, le maquillage, du fait du plissement des paupières, a tendance à perdre son uniformité et à se concentrer plus ou moins rapidement et profondément dans les plis de la paupière. Il en résulte un effet jugé généralement peu esthétique.

Les fards à paupières anhydres, et notamment ceux se présentant sous forme de poudre compacte, après leur application sur les paupières, ont tendance à s'effriter au cours du temps, notamment en raison de l'action mécanique exercée par le clignement des paupières sur le maquillage déposé. Il s'ensuit une perte de matière engendrant une détérioration du maquillage et nécessitant à l'utilisatrice de renouveller l'application du maquillage.

Par ailleurs, pour les fards à paupières aqueux qui ont une texture fluide, les matières colorantes peuvent décanter pendant le stockage, cette instabilité rendant alors le produit de maquillage inadapté pour une application homogène sur les paupières.

Le but de la présente invention est de disposer d'un fard à paupières ayant une texture fluide et présentant une bonne stabilité au stockage, notamment pendant 2 mois à 45 °C.

Un autre but de l'invention est de disposer d'un fard à paupières qui s'étale facilement sur les paupières et forme sur celle-ci un maquillage homogène, présentant une bonne tenue dans le temps.

Les inventeurs ont découvert qu'un tel fard à paupières pouvait être obtenu en utilisant dans un mileu aqueux de la gomme de xanthane, un silicate mixte et une matière colorante. Avec ces gélifiants, le fard à paupières est fluide (il s'écoule sous son propore poids à la température ambiante de 25 °C) et le gel reste bien homogène au cours du temps, notamment au stockage pendant deux semaines à 45 °C : Ce gel est bien compatible avec les matières colorantes, notamment avec les pigments. Le fard à paupière présente donc une bonne stabilité dans le temps.

Il est connu des documents US 4604281, US 4994264 et JP 57171907 des compositions cosmétiques comprenant des matières colorantes, une gomme de xanthane et un silicate mixte mais ces documents ne prévoient nullement l'incorporation du silicate mixte et de la gomme de xanthane dans un ratio pondéral silicate mixte / gomme de xanthane allant de 8 à 13 ni la présence d'un polyol spécifique pour obtenir une bonne dispersion des matières colorantes et éviter leur décantation pendant le stockage

Par ailleurs, le fard à paupières s'étale bien sur les paupières, procure une agréable sensation de fraîcheur et le maquillage obtenu présente une répartition homogène des matières colorantes. De plus, le maquillage présente une bonne tenue dans le temps au sébum et/ou à l'action mécanique du mouvement (clignement) des paupières : les matières colorantes ne diffusent pas dans les plis des paupières et restent donc bien réparties sur les paupières au cours du temps. Le maquillage se maintient bien sur les paupières et ne s'effrite pas. En outre, le fard à paupières se démaquille facilement à l'eau sans nécessité d'employer un démaquillant spécifique.

De façon plus précise, l'invention a pour objet un fard à paupières comprenant, dans un milieu aqueux cosmétiquement acceptable, au moins une gomme de xanthane, un silicate mixte et une matière colorante présente en une teneur supérieure ou égale à 10,1 % en poids, par rapport au poids total du fard à paupières et un glycol comprenant de 2 à 8 atomes de carbone, ledit silicate mixte étant une laponite.

L'invention a aussi pour objet un procédé cosmétique de maquillage des paupières, comprenant l'application sur les paupières, du fard à paupières tel que défini précédemment.

L'invention a également pour objet l'utilisation d'un fard à paupières tel que défini précédemment pour l'obtention d'un maquillage, déposé sur les paupières, homogène et/ou présentant une bonne tenue.

L'invention a encore pour objet l'utilisation de gomme de xanthane, de silicate mixte et de glycol comprenant de 2 à 8 atomes de carbone, ledit silicate étant une laponite dans un fard à paupières contenant, dans un milieu aqueux cosmétiquement acceptable, une matière colorante en une teneur supérieure ou égale à 10,1 % en poids, par rapport au poids total du fard à paupières, pour l'obtention d'un maquillage déposé sur les paupières, homogène et/ou présentant une bonne tenue.

On entend par silicate mixte les silicates d'origine naturelle ou synthétique renfermant plusieurs (deux ou plus) types de cations choisis parmi les métaux alcalins (par exemple Na, Li, K) ou alcalino-terreux (par exemple Be, Mg, Ca), les métaux de transition et l'aluminium.

Pour garantir de bonnes propriétés cosmétiques, ces silicates doivent se présenter de préférence sous une forme finement divisée, et en particulier sous forme de particules ayant une taille moyenne allant de 5 nm à 1000 nm, et de préférence de 20 nm à 600 nm. Les particules de silicates mixtes ont généralement la forme de disques ou de feuillets. Aussi, on entend ici par taille moyenne de particules, la taille moyenne en nombre de la plus grande dimension (longueur) de ces disques ou feuillets. Ces particules sous forme de disques ou feuillets ont généralement une épaisseur allant d'environ 0,5 à 5 nm.

On utilise dans la présente invention des phyllosilicates, à savoir des silicates ayant une structure dans laquelle les tétraèdres SiO₄ sont organisés en feuillets entre lesquels se trouvent enfermés les cations métalliques.

Les laponites sont des silicates de magnésium, de sodium et éventuellement de lithium, ayant une structure en couches semblable à celle des montmorillonites. La laponite est la forme synthétique du minéral naturel appelé "hectorite". L'origine synthétique de cette famille de silicates présente un avantage considérable par rapport à la forme naturelle car elle permet une bonne maîtrise de la composition du produit. En outre, les laponites ont l'avantage d'avoir une taille de particules bien inférieure à celles de l'hectorite et de la bentonite naturelles.

Comme laponites, on peut citer notamment les produits vendus par la société Laporte sous le nom Laponite XLS, Laponite XLG, Laponite RD, Laponite RDS par la société ROCKWOOD (ces produits sont des silicates de sodium et de magnésium et des silicates de sodium, de lithium et de magnésium).

Le silicate mixte peut être présent dans la composition selon l'invention en une teneur allant de 0,1 % à 10 % en poids, par rapport au poids total de la composition, de préférence allant de 0,5 % à 5 % en poids, et préférentiellement allant de 1 % à 4 % en poids.

Les gommes de xanthanes sont généralement synthétisées par fermentation de sucres par la bactérie XANTHOMONAS CAMPESTRIS et les mutants ou variantes de celle-ci.

Les gommes de xanthane comportent dans leur structure 3 monosaccharides différents qui sont le mannose, le glucose et l'acide glucuronique sous forme de sel, notamment de sel de sodium.

Les gommes de xanthane ont généralement une viscosité allant de 0,6 à 1,65 Pa.s. (mesurée à 25 °C au viscosimètre Brookfield, type LVT à 60 t/min). Elles ont un poids moléculaire pouvant aller de 1 000 000 à 50 000 000.

De tels produits sont plus particulièrement : le KELTROL T vendu par la société KELTRO, le RHODICARE S vendu par la société RHODIA, le KELSAN S vendu par la société MONSANTO CHEMICAL.

La gomme de xanthane peut être présente dans la composition selon l'invention en une teneur allant de 0,01 % à 10 % en poids, par rapport au poids total de la composition, de préférence allant de 0,1 % à 5 % en poids, et préférentiellement allant de 0,1 % à 3 % en poids.

Avantageusement, le silicate mixte et la gomme de xanthane sont présents en une teneur telle que le rapport pondéral silicate mixte / gomme de xanthane va de 0,1 à 20, de préférence va de 5 à 15, et préférentiellement va de 8 à 13.

La composition selon l'invention comprend un mileu aqueux cosmétiquement acceptable, c'est-à-dire un milieu aqueux compatibles avec la peau des paupières. En particulier, ce milieu aqueux forme une phase aqueuse continue. La composition peut comprendre en particulier de l'eau en une teneur supérieure ou égale à 20 % en poids, notamment allant de 20 % à 89,7 % en poids, par rapport au poids total de la composition, de préférence allant de 35 % à 89,7 % en poids, et préférentiellement allant de 40 % à 89,7 % en poids.

La phase aqueuse peut comprendre en outre un solvant miscible à l'eau comme par exemple des monoalcools en C₂-C₆ tels que l'éthanol, l'isopropanol, le n-propanol. Le solvant organique miscible à l'eau, tels que les monoalcools en C₂-C₆, peut être présent en une teneur allant de 0,1 % à 20 % en poids, par rapport au poids total de la composition, et de préférence allant de 0,5 % à 15 % en poids.

La composition comprenant une phase aqueuse, elle se présente de préférence sous forme d'une composition fluide, c'est à dire sous forme d'une composition qui s'écoule sous son propre poids, à la température ambiante (25 °C). La composition peut notamment se présenter sous forme de gel aqueux.

Selon une variante de réalisation de l'invention, la composition peut comprendre en outre une phase grasse dispersée dans la phase aqueuse pour former une émulsion huile-dans-eau.

La phase grasse comprend au moins une huile, qui peut être choisie parmi les huiles d'origine minérale, animale, végétale ou synthétique, hydrocarbonées et/ou siliconées, seules ou en mélange.

Comme exemple d'huiles hydrocarbonées, on peut citer l'huile de paraffine ou de vaseline, l'huile de vison, de tortue, de soja, le perhydrosqualène, l'huile d'amande douce, de calophyllum, de palme, de pépins de raisin, de sésame, de maïs, d'arara, de colza, de tournesol, de coton, d'abricot, de ricin, d'avocat, de jojoba, d'olive ou de germes de céréales ; des esters d'acide lanolique, d'acide oléique, d'acide laurique, d'acide stéarique ; les esters gras, tels que le myristate d'isopropyle, le palmitate d'isopropyle, le stéarate de butyle, le laurate d'hexyle, l'adipate de diisopropyle, l'isononanoate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéarate de glycérine ou de diglycérine ; les acides gras supérieurs tels que l'acide myristique, l'acide palmitique, l'acide stéarique, l'acide béhénique, l'acide oléique, l'acide linoléique, l'acide linolénique ou l'acide isostéarique ; les alcools gras supérieurs tels que le cétanol, l'alcool stéarylique ou l'alcool oléique, l'alcool linoléique ou linolénique, l'alcool isostéarique ou l'octyl dodécanol.

Comme huiles siliconées, on peut citer par exemple les polydiméthylsiloxanes (PDMS) volatiles ou non à chaîne siliconée linéaire ou cyclique, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ; leurs mélanges.

L'huile peut être présente dans la composition selon l'invention en une teneur allant de 0,1 % à 30 % en poids, par rapport au poids total de la composition, de préférence allant de 1 % à 20 % en poids, et préférentiellement allant de 5 % à 15 % en poids.

La phase grasse peut comprendre en outre d'autres corps gras additionnels autres que les huiles, tels que les cires, les pâteux ou les gommes.

Comme cires (corps gras solide à température ambiante), on peut citer les cires hydrocarbonées telles que la cire d'abeilles éventuellement modifiée, la cire de Carnauba, de Candellila, d'Ouricoury, du Japon, les cires de fibres de liège ou de canne à sucre, les cires de paraffine, de lignite, les cires microcristallines, la cire de lanoline, la cire de Montan, les ozokérites, les cires de polyéthylène, les cires obtenues par synthèse de Fischer-Tropsch et les alcools gras en C₂₀-C₆₀. On peut également utiliser des cires de silicone, parmi lesquelles on peut citer les alkyl, alcoxy et/ou esters de polyméthylsiloxane et leurs mélanges.

Comme corps gras pâteux, on peut citer des corps gras ayant un point de fusion allant de 25 à 45 °C et/ou une viscosité à 40 °C allant de 0,1 à 40 Pa.s mesurée au Contraves TV équipé d'un mobile MS-r3 ou Ms-r4 tournant à 60 Hz. A titre d'exemple de corps gras pâteux, on peut citer les lanolines et les dérivés de lanoline comme les lanolines acétylées ou les lanolines oxypropylènées, ayant une viscosité de 18 à 21 Pa.s, de préférence 19 à 20,5 Pa.s, et/ou un point de fusion de 30 à 55°C et leurs mélanges. On peut également utiliser des esters d'acides ou d'alcools gras, notamment ceux ayant 20 à 65 atomes de carbone (point de fusion de l'ordre de 20 à 35°C et/ou viscosité à 40 °C allant de 0,1 à 40 Pa.s) comme le citrate de tri-isostéaryle ou de cétyle ; le propionate d'arachidyle ; le polylaurate de vinyle ; les esters du cholestérol comme les triglycérides d'origine végétale tels que les huiles végétales hydrogénées, les polyesters visqueux comme l'acide poly(12-hydroxystéarique) et leurs mélanges. Comme triglycérides d'origine végétale, on peut utiliser les dérivés d'huile de ricin hydrogénée, tels que le « THIXINR » de Rheox.
On peut aussi citer les corps gras pâteux siliconés tels que les polydiméthylsiloxanes (PDMS) ayant des chaînes pendantes du type alkyle ou alcoxy ayant de 8 à 24 atomes de carbone, et un point de fusion de 20-55°C, comme les stearyl dimethicones notamment ceux vendus par la société Dow Corning sous les noms commerciaux de DC2503 et DC25514, et leurs mélanges.

Comme gommes, on peut utiliser les gommes de silicone (diméthiconol).

Le corps gras additionnel peut être présent en une teneur allant de 0,1 % à 25 % en poids, par rapport au poids total de la composition, et de préférence allant de 1 % à 15% en poids.

Lorsque la composition est sous forme d'émulsion huile-dans-eau, elle peut comprendre un émulsionnant qui est en particulier un émulsionnant choisi de manière appropriée pour l'obtention d'émulsion huile-dans-eau. En particulier, on utilise un émulsionnant hydrosoluble, en particulier ayant une balance HLB (hydrophilelipophile balance) supérieure ou égale à 10.

L'émulsionnant peut être choisi parmi les émulsionnants amphotères, anioniques, cationiques ou non ioniques, et leurs mélanges.
- comme émulsionnants amphotères, on peut citer les N-acyl-aminoacides tels que les N-alkyl-aminoacétates et le cocoamphodiacetate disodique et les oxydes d'aminés tels que l'oxyde de stéaramine ;
- comme émulsionnants anioniques, on peut citer les acylglutamates tels que le "disodium hydrogenated tallow glutamate" (AMISOFT HS-21^{R} commercialisé par la société Ajinomoto) ; les acides carboxyliques et leurs sels tels que le stéarate de sodium ; les esters phophoriques et leurs sels tels que le "DEA oleth-10 phosphate" ; les sulfosuccinates tels que le "Disodium PEG-5 citrate lauryl sulfosuccinate" et le "Disodium ricinoleamido MEA sulfosuccinate" ; les alkyl éther sulfates tels que le lauryl éther sulfate de sodium ; les sulfosuccinates; les iséthionates.
- comme émulsionnants cationiques, on peut citer les alkyl-imidazolidinium tels que l'étho-sulfate d'isostéaryl-éthylimidonium ; les sels d'ammonium tels que le chlorure de N,N,N-triméthyl-1-docosanaminium (Behentrimonium chloride) ;
- comme émulsionnants non ioniques, on peut citer les esters et éthers d'oses tels que le stéarate de sucrose, le cocoate de sucrose, et le mélange de stéarate de sorbitan et de cocoate de sucrose commercialisé par la société ICI sous la dénomination d'Arlatone 2121 ; les esters d'acides gras (notamment d'acide en C8-C24, et de préférence en C16-C22) et de polyol, notamment de glycérol ou de sorbitol, tels que le stéarate de glycéryle, le stéarate de polyglycéryl-2, le tristéarate de sorbitan, le ricinoléate de glycéryle ; les éthers de glycérol ; les éthers oxyéthylénés et/ou oxypropylénés (pouvant comporter de 1 à 150 groupes oxyéthylénés et/ou oxypropylénés) d'alcools gras (notamment d'alcool en C8-C24, et de préférence en C12-C18) tels que l'éther oxyéthyléné, oxypropyléné de l'alcool laurique à 25 groupes oxyéthylénés et 25 groupes oxypropylénés (nom CTFA "PPG-25 laureth-25") et l'éther oxyéthylèné du mélange d'alcools gras en C12-C15 comportant 7 groupes oxyéthylénés (nom CTFA "C12-15 Pareth-7") ; les esters d'acide gras (notamment d'acide en C8-C24, et de préférence en C16-C22) et de polyéthylène glycol (pouvant comprendre de 1 à 150 motifs d'éthylèneglycol) tels que le stéarate de PEG-50 et le stéarate de PEG-40 ; les copolymères d'oxyde de propylène et d'oxyde d'éthylène tels que ceux vendus sous les dénominations « SYNPERONIC PE/F68 », « SYNPERONIC PE/L44 », « SYNPERONIC PE/F127 » par la société UNIQEMA.
- comme émulsionnants siliconés, on peut citer les diméthicone copolyols., telle que celles vendues sous les dénominations "DC2-5695" et « Q2-5220 » par la société Dow Corning, le mélange de cyclomethicone/dimethicone copolyol vendu sous la dénomination "Q2-3225C" par la société Dow Corning, les diméthicone copolyols phospates tels que celui vendu sous la dénomination « PECOSIL PS 100» par la société PHOENIX CHEMICAL. Comme émulsionnant siliconé, on peut également utiliser un diméthicone copolyol benzoate c'est-à-dire un ester partiel d'acide benzoïque et de diméthicone copolyol, ce dernier étant un polymère de diméthylpolysiloxane comportant des chaînes latérales de polyoxyéthylène et/ou de polyoxypropylène. Comme diméthicone copolyol benzoate, on peut utiliser ceux vendus sous la dénomination « FINSOLV^{®} SLB-101 », « FINSOLV^{®} SLB-201 » par la société FINETEX.

L'émulsionnant peut être présent dans la composition selon l'invention en une teneur allant de 0,1 % à 10 % en poids, par rapport au poids total de la composition, de préférence allant de 0,5 % à 8 % en poids, et préférentiellement allant de 0,5 % à 5 % en poids.

La matière colorante présente dans la composition selon l'invention peut être choisie parmi les matières colorantes pulvérulentes comme les pigments, les nacres, les paillettes ou bien encore les matières colorantes hydrosolubles, habituellement utilisés dans les compositions cosmétiques, et leurs mélanges.

Les pigments peuvent être blancs ou colorés, minéraux et/ou organiques, interférentiels ou non. On peut citer, parmi les pigments minéraux, le dioxyde de titane, éventuellement traité en surface, les oxydes de zirconium ou de cérium, ainsi que les oxydes de zinc, de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants hydrosolubles sont par exemple le jus de betterave, le bleu de méthylène, le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle.

La matière colorante est présente dans la composition selon l'invention en une teneur supérieure ou égale à 10,1 % en poids, par rapport au poids total de la composition, et notamment va de 10,1 % à 50 % en poids, de préférence va de 10,1 % à 40 % en poids, et préférentiellement va de 15 % à 30 % en poids.

La composition selon l'invention contient, en outre, au moins un glycol pour permettre un bon mouillage des pigments, c'est-à-dire faciliter leur mise en oeuvre et leur dispersion homogène (absence d'agglomérat) dans le milieu aqueux de la composition. Le glycol permet un bon mouillage de la peau facilitant l'étalement de la composition sur la paupière. Dans la présente demande, on entend par glycol un diol comprenant de 2 à 8, et de préférence de 2 à 4, atomes de carbone.

Le glycol peut être choisi parmi le propylène glycol, l'éthylène glycol, le 1,3-butylène glycol, le dipropylène glycol.

Le glycol peut être présent dans la composition en une teneur allant de 0,1 % à 30 % en poids, par rapport au poids total de la composition, et de préférence de 5 % à 20 % en poids.

La composition selon l'invention peut comprendre des charges qui peuvent être choisies parmi celles bien connues de l'homme du métier et couramment utilisées dans les compositions cosmétiques. Comme charge, on peut notamment utiliser :
- le talc qui est un silicate de magnésium hydraté utilisé sous forme de particules généralement inférieures à 40 microns,
- les micas qui sont des aluminosilicates de compositions variées se présentant sous la forme d'écailles ayant des dimensions de 2 à 200 microns, de préférence de 5 à 70 microns et une épaisseur comprise entre 0,1 à 5 microns, de préférence de 0,2 à 3 microns, ces micas pouvant être d'origine naturelle telle que la muscovite la margarite, la roscoelithe, la lipidolithe, la biotite ou d'origine synthétique,
- l'amidon en particulier l'amidon de riz,
- le kaolin qui est un silicate d'aluminium hydraté qui se présente sous la forme de particules de forme isotrope ayant des dimensions généralement inférieures à 30 microns,
- les oxydes de zinc et de titane généralement utilisés sous la forme de particules ayant des dimensions ne dépassant pas quelques microns,
- le carbonate de calcium, le carbonate ou l'hydrocarbonate de magnésium,
- la cellulose microcristalline,
- la silice,
- les poudres de polymères synthétiques tels que le polyéthylène, les polyesters (l'isophtalate ou le téréphtalate de polyéthylène), les polyamides tels que ceux vendus sous la dénomination commerciale de "Nylon" ou de "Téflon" et les poudres de silicone.

La composition selon l'invention peut comprendre au moins un additif cosmétique choisi dans le groupe formé par les conservateurs, les parfums, les vitamines, les agents hydratants, les agents adoucissants, les filtres solaires, les polymères filmogènes, les séquestrants, les agents alcalinisants ou acidifiants.

Bien entendu, l'homme du métier veillera à choisir ce ou ces éventuels composés complémentaires, et/ou leur quantité, de manière telles que les propriétés avantageuses de la composition selon l'invention ne soient pas, ou substantiellement pas, altérées par l'adjonction envisagée.

La composition selon l'invention peut être préparée selon les méthodes usuelles des domaines considérés.

L'invention est illustrée plus en détail dans les exemples suivants.

### Exemple 1 :

- Gomme de xanthane 0,3 g
- Silicate de sodium et de magnesium (Laponite XLG de la société ROCKWOOD) 3 g
- Ethanol 10 g
- Propylène glycol 7 g
- Glycérine 5 g
- Diméthicone copolyol benzoate (FINSOLV SLB-101 de la société FINETEX) 3 g
- Pigments 10 g
- Oxychlorure de bismuth 5 g
- Conservateurs qs
- Eau qsp 100 g

Ce fard à paupières fluide est stable au stockage pendant 2 mois à 45 °C. Il s'étale facilement sur les paupières en procurant une sensation de fraîcheur et forme un maquillage homogène ne formant pas de stries et présentant une bonne tenue, en particulier une bonne résistance au sébum et à l'action mécanique (clignement) des paupières. Il se démaquille aisément avec de l'eau.

### Exemple 2 :

On a préparé un fard à paupières ayant la composition suivante :
- Gomme de xanthane 0,3 g
- Silicate de sodium et de magnésium (Laponite XLG de la société ROCKWOOD) 3 g
- Ethanol 10 g
- Propylèneglycol 7 g
- Glycérine 5 g
- Silicone phénylée (BELSIL PDM 1000 de la société WACKER) 10g
- Diméthicone copolyol benzoate (FINSOLV SLB-101 de la société FINETEX) 3 g
- Pigments 10 g
- Oxychlorure de bismuth 5 g
- Conservateurs qs
- Eau qsp 100 g

Ce fard à paupières fluide est stable au stockage pendant 2 mois à 45 °C. Il s'applique facilement sur les paupières en procurant une sensation de fraîcheur et forme un maquillage homogène et de bonne tenue.

### Exemple 3 :

On a préparé un fard à paupières ayant la composition suivante :
- Gomme de xanthane 0,3 g
- Silicate de sodium et de magnesium (Laponite XLG de la société ROCKWOOD) 3 g
- Ethanol 10 g
- Glycérine 5g
- Propylène glycol 7 g
- Mélange de polydiméthylsiloxane à groupements αω-hydroxyle et de cyclopentadiméthylsiloxane (15/85) (DC2-9071 de DOW CORNING) 10 g
- Diméthicone copolyol benzoate (FINSOLV SLB-101 de la société FINETEX) 3g
- Pigments 15 g
- Oxychlorure de bismuth 5 g
- Conservateurs qs
- Eau qsp 100 g

Ce fard à paupières forme un maquillage homogène sur les paupières et résistant aux mouvements des paupières.

### Exemple 4 :

On a préparé un fard à paupières ayant la composition suivante :
- Gomme de xanthane 0,3 g
- Silicate de sodium et de magnésium (Laponite XLG de la société ROCKWOOD) 3 g
- Ethanol 10 g
- Propylène glycol 7 g
- Glycérine 5 g
- Silicone phénylée (BELSIL PDM 1000 de la société WACKER) 10 g
- Diméthicone copolyol (Q2-5220 de DOW CORNING) 3 g
- Pigments 15 g
- Oxychlorure de bismuth 5 g
- Conservateurs qs
- Eau qsp 100 g

Ce fard à paupières s'applique facilement sur les paupières et forme un maquillage homogène, de bonne tenue.

### Exemple 5 :

On a préparé un fard à paupières ayant la composition suivante :
- Gomme de xanthane 0,3 g
- Silicate de sodium et de magnesium (Laponite XLG de la société ROCKWOOD) 3 g
- Ethanol 10 g
- Propylène glycol 7 g
- Glycérine 5 g
- Isononanoate d'isononyle 10 g
- Diméthicone copolyol benzoate (FINSOLV SLB-101 de la société FINETEX) 3 g
- Pigments 15 g
- Oxychlorure de bismuth 5 g
- Conservateurs qs
- Eau qsp 100 g

Ce fard à paupières s'applique facilement sur les paupières et forme un maquillage homogène, de bonne tenue.

### Exemple 6 :

On a préparé un fard à paupières ayant la composition suivante :

| | |
|---|---|
| Gomme de xanthane | 0,3 g |
| Silicate de sodium et de magnesium (Laponite XLG de la société ROCKWOOD) | 3 g |
| Ethanol | 10 g |
| Propylène glycol | 7 g |
| Glycérine | 5 g |
| Isononanoate d'isononyle | 10 g |
| Stéarate de polyéthylène glycol (40 OE) | 5 g |
| Pigments | 15 g |
| Oxychlorure de bismuth | 5 g |
| Conservateurs qs | |
| Eau qsp | 100 g |

Ce fard à paupières s'applique facilement sur les paupières et forme un maquillage homogène, de bonne tenue.

## Revendications

1. Fard à paupières comprenant dans un milieu aqueux cosmétiquement acceptable, une gomme de xanthane, un silicate mixte, une matière colorante présente en une teneur supérieure ou égale à 10,1 % en poids, par rapport au poids total de la composition et un glycol comprenant de 2 à 8 atomes de carbone, ledit silicate mixte étant une laponite.

2. Fard à paupières selon la revendication précédente, **caractérisé par le fait que** le silicate mixte se présente sous une forme finement divisée ayant une taille moyenne de particules allant de 15 nm à 1000 nm.

3. Fard à paupières selon l'une quelconque des revendications précédentes, **caractérisé par le fait que** le silicate mixte est présent en une teneur allant de 0,1 % à 10 % en poids, par rapport au poids total de la composition, de préférence allant de 0,5 % à 5 % en poids, et préférentiellement allant de 1 % à 4 % en poids.

4. Fard à paupières selon l'une quelconque des revendications précédentes, **caractérisé par le fait que** la gomme de xanthane est présente en une teneur allant de 0,01 % à 10 % en poids, par rapport au poids total de la composition, de préférence allant de 0,1 % à 5 % en poids, et préférentiellement allant de 0,1 % à 3 % en poids.

5. Fard à paupières selon l'une quelconque des revendications 1 à 4, **caractérisé par le fait que** le silicate mixte et la gomme de xanthane sont présents en une teneur telle que le rapport pondéral silicate mixte / gomme de xanthane va de 0,1 à 20, de préférence va de 5 à 15, et préférentiellement va de 8 à 13.

6. Fard à paupières selon l'une quelconque des revendications précédentes, **caractérisé par le fait qu'**elle comprend au moins 20 % en poids, par rapport au poids total de la composition, d'eau.

7. Fard à paupières selon la revendication précédente, **caractérisé par le fait que** l'eau est présente en une teneur allant de 20 % à 89,7 % en poids, par rapport au poids total de la composition, de préférence allant de 35 % à 89,7 % en poids, et préférentiellement allant de 40 % à 89,7 % en poids.

8. Fard à paupières selon l'une quelconque des revendications précédentes, **caractérisé par le fait qu'**il comprend un monoalcool en C₂-C₆ tel que l'éthanol, l'isopropanol, le n-propanol.

9. Fard à paupières selon la revendication précédente, **caractérisé par le fait que** le monoalcool en C₂-C₆, est présent en une teneur allant de 0,1 % à 20 % en poids, par rapport au poids total de la composition, et de préférence allant de 0,5 % à 15 % en poids.

10. Fard à paupières selon l'une quelconque des revendications précédentes, **caractérisé par le fait que** la matière colorante est choisie dans le groupe formé par les pigments, les nacres, les paillettes, les matières colorantes hydrosolubles, et leurs mélanges.

11. Fard à paupières selon l'une quelconque des revendications précédentes, **caractérisé par le fait que** la matière colorante est choisie dans le groupe formé par le dioxyde de titane, les oxydes de zirconium ou de cérium, les oxydes de zinc, de fer ou de chrome, le violet de manganèse, le bleu outremer, l'hydrate de chrome, le bleu ferrique, le noir de carbone, les laques à base de carmin de cochenille, de baryum, strontium, calcium, aluminium ; le mica recouvert de titane ou d'oxychlorure de bismuth, le mica titane avec des oxydes de fer, le mica titane avec du bleu ferrique ou de l'oxyde de chrome, les pigments nacrés à base d'oxychlorure de bismuth.

12. Fard à paupières selon l'une quelconque des revendications précédentes, **caractérisé par le fait que** la matière colorante est présente en une teneur et notamment allant de 10,1 % à 50 % en poids, par rapport au poids total de la composition, de préférence allant de 10,1 % à 40 % en poids, et préférentiellement allant de 15 % à 30 % en poids.

13. Fard à paupières selon l'une des revendications précédentes, **caractérisé par le fait que** le glycol est choisi parmi le propylène glycol, l'éthylène glycol, le 1,3-butylène glycol, le dipropylène glycol.

14. Fard à paupières selon l'une des revendications précédentes, **caractérisé par le fait que** le glycol est présent en une teneur allant de 0,1 % à 30 % en poids, par rapport au poids total de la composition, et de préférence de 5 % à 20 % en poids.

15. Fard à paupières selon l'une quelconque des revendications précédentes, **caractérisé par le fait qu'**il se présente sous forme d'émulsion huile-dans-eau.

16. Fard à paupières selon la revendication précédente, **caractérisé par le fait qu'**il comprend une phase grasse comprenant au moins une huile.

17. Fard à paupières selon la revendication précédente, **caractérisé par le fait que** l'huile est choisi dans le groupe formée par les huiles hydrocarbonées et les huiles siliconées.

18. Fard à paupières selon la revendication 16 ou 17, **caractérisé par le fait que** l'huile est présente en une teneur allant de 0,1 % à 30 % en poids, par rapport au poids total de la composition, de préférence allant de 1 % à 20 % en poids, et préférentiellement allant de 5 % à 15 % en poids.

19. Fard à paupières selon l'une quelconque des revendications 16 à 18, **caractérisé par le fait que** la phase grasse comprend un corps gras additionnel choisi parmi les cires, les gommes et les pâteux.

20. Fard à paupières selon la revendication précédente, **caractérisé par le fait que** le corps gras additionnel est présent en une teneur allant de 0,1 % à 25 % en poids, par rapport au poids total de la composition, et de préférence allant de 1 % à 15 % en poids.

21. Fard à paupières selon l'une quelconque des revendications 16 à 19, **caractérisé par le fait qu'**il comprend un émulsionnant.

22. Fard à paupières selon la revendication précédente, **caractérisé par le fait que** l'émulsionnant est choisi dans le groupe formé par les N-acyl-aminoacides, les oxydes d'aminés, les acylglutamates, les acides carboxyliques et leurs sels, les esters phophoriques et leurs sels, les sulfosuccinates, les alkyl éther sulfates, les sulfosuccinates, les iséthionates, les alkyl-imidazolidinium; les sels d'ammonium; les esters et éthers d'oses, les esters d'acides gras et de polyol, les éthers de glycérol, les éthers oxyéthylénés et/ou oxypropylénés d'alcools gras, les esters d'acide gras et de polyéthylène glycol, les copolymères d'oxyde de propylène et d'oxyde d'éthylène, les diméthicone copolyols, les diméthicone copolyols phosphate, les diméthicone copolyols benzoate.

23. Fard à paupières selon la revendication 21 ou 22, **caractérisé par le fait que** l'émulsionnant est présent en une teneur allant de 0,1 % à 10 % en poids, par rapport au poids total de la composition, de préférence allant de 0,5 % à 8 % en poids, et préférentiellement allant de 0,5 % à 5 % en poids.

24. Fard à paupières selon l'une quelconque des revendications précédentes, **caractérisé par le fait qu'**il comprend un additif cosmétique choisi dans le groupe formé par les charges, les conservateurs, les parfums, les vitamines, les agents hydratants, les agents adoucissants, les filtres solaires, les polymères filmogènes, les séquestrants, les agents alcalinisants ou acidifiants.

25. Procédé de maquillage des paupières comprenant l'application sur les paupières d'un fard à paupières selon l'une quelconque des revendications précédentes.

26. Utilisation d'un fard à paupière selon l'une quelconque des revendications 1 à 24 pour l'obtention d'un maquillage, déposé sur les paupières, homogène et/ou présentant une bonne tenue.

27. Utilisation de gomme de xanthane, d'un silicate mixte ledit silicate mixte étant une laponite et un glycol comprenant de 2 à 8 atomes de carbone dans un fard à paupières contenant, dans un milieu aqueux cosmétiquement acceptable, une matière colorante en une teneur supérieure ou égale à 10,1 % en poids, par rapport au poids total du fard à paupières, pour l'obtention d'un maquillage déposé sur les paupières, homogène et/ou présentant une bonne tenue.

## Claims

1. Eyeshadow comprising, in a cosmetically acceptable aqueous medium, a xanthan gum, a mixed silicate, a dyestuff present in a content of greater than or equal to 10.1% by weight, relative to the total weight of the composition, and a glycol containing from 2 to 8 carbon atoms, the said mixed silicate being a laponite.

2. Eyeshadow according to the preceding claim, **characterized in that** the mixed silicate is in a finely divided form with a mean particle size ranging from 15 nm to 1000 nm.

3. Eyeshadow according to either of the preceding claims, **characterized in that** the mixed silicate is present in a content ranging from 0.1% to 10% by weight, preferably ranging from 0.5% to 5% by weight and preferentially ranging from 1% to 4% by weight, relative to the total weight of the composition.

4. Eyeshadow according to any one of the preceding claims, **characterized in that** the xanthan gum is present in a content ranging from 0.01% to 10% by weight, preferably ranging from 0.1% to 5% by weight and preferentially ranging from 0.1% to 3% by weight, relative to the total weight of the composition.

5. Eyeshadow according to any one of Claims 1 to 4, **characterized in that** the mixed silicate and the xanthan gum are present in a content such that the mixed silicate/xanthan gum weight ratio ranges from 0.1 to 20, preferably from 5 to 15 and preferentially from 8 to 13.

6. Eyeshadow according to any one of the preceding claims, **characterized in that** it comprises at least 20% by weight of water, relative to the total weight of the composition.

7. Eyeshadow according to the preceding claim, **characterized in that** the water is present in a content ranging from 20% to 89.7% by weight, preferably ranging from 35% to 89.7% by weight and preferentially ranging from 40% to 89.7% by weight, relative to the total weight of the composition.

8. Eyeshadow according to any one of the preceding claims, **characterized in that** it comprises a C₂-C₆ monoalcohol such as ethanol, isopropanol or n-propanol.

9. Eyeshadow according to the preceding claim, **characterized in that** the C₂-C₆ monoalcohol is present in a content ranging from 0.1% to 20% by weight, and preferably ranging from 0.5% to 15% by weight, relative to the total weight of the composition.

10. Eyeshadow according to any one of the preceding claims, **characterized in that** the dyestuff is chosen from the group formed by pigments, nacres, flakes and water-soluble dyestuffs, and mixtures thereof.

11. Eyeshadow according to any one of the preceding claims, **characterized in that** the dyestuff is chosen from the group formed by titanium dioxide, zirconium oxide or cerium oxide, zinc oxide, iron oxide, chromium oxide, manganese violet, ultramarine blue, chromium hydrate, ferric blue, carbon black, lakes based on cochineal carmine or on barium, strontium, calcium or aluminium; mica coated with titanium or with bismuth oxychloride, titanium mica with iron oxides, titanium mica with ferric blue or chromium oxide, and nacreous pigments based on bismuth oxychloride.

12. Eyeshadow according to any one of the preceding claims, **characterized in that** the dyestuff is present in a content especially ranging from 10.1% to 50% by weight, preferably ranging from 10.1% to 40% by weight and preferentially ranging from 15% to 30% by weight, relative to the total weight of the composition.

13. Eyeshadow according to one of the preceding claims, **characterized in that** the glycol is chosen from propylene glycol, ethylene glycol, 1,3-butylene glycol and dipropylene glycol.

14. Eyeshadow according to one of the preceding claims, **characterized in that** the glycol is present in a content ranging from 0.1% to 30% by weight and preferably from 5% to 20% by weight, relative to the total weight of the composition.

15. Eyeshadow according to any one of the preceding claims, **characterized in that** it is in the form of an oil-in-water emulsion.

16. Eyeshadow according to the preceding claim, **characterized in that** it comprises a fatty phase comprising at least one oil.

17. Eyeshadow according to the preceding claim, **characterized in that** the oil is chosen from the group formed by hydrocarbon-based oils and silicone oils.

18. Eyeshadow according to claim 16 or 17, **characterized in that** the oil is present in a content ranging from 0.1% to 30% by weight, preferably ranging from 1% to 20% by weight and preferentially ranging from 5% to 15% by weight, relative to the total weight of the composition.

19. Eyeshadow according to any one of claims 16 to 18, **characterized in that** the fatty phase comprises an additional fatty substance chosen from waxes, gums and pasty substances.

20. Eyeshadow according to the preceding claim, **characterized in that** the additional fatty substance is present in a content ranging from 0.1% to 25% by weight and preferably ranging from 1% to 15% by weight, relative to the total weight of the composition.

21. Eyeshadow according to any one of Claims 16 to 19, **characterized in that** it comprises an emulsifier.

22. Eyeshadow according to the preceding claim, **characterized in that** the emulsifier is chosen from the group formed by N-acylamino acids, amine oxides, acylglutamates, carboxylic acids and salts thereof, phosphoric esters and salts thereof, sulfosuccinates, alkyl ether sulfates, sulfosuccinates, isethionates, alkylimidazolidiniums; ammonium salts; saccharide esters and ethers, fatty acid esters of polyols, glycerol ethers, oxyethylenated and/or oxypropylenated ethers of fatty alcohols, fatty acid esters of polyethylene glycol, copolymers of propylene oxide and of ethylene oxide, dimethicone copolyols, dimethicone copolyol phosphates and dimethicone copolyol benzoates.

23. Eyeshadow according to Claim 21 or 22, **characterized in that** the emulsifier is present in a content ranging from 0.1% to 10% by weight, preferably ranging from 0.5% to 8% by weight and preferentially ranging from 0.5% to 5% by weight, relative to the total weight of the composition.

24. Eyeshadow according to any one of the preceding claims, **characterized in that** it comprises a cosmetic additive chosen from the group formed by fillers, preserving agents, fragrances, vitamins, moisturizers, softeners, sunscreens, film-forming polymers, sequestrants and acidifying or basifying agents.

25. Process for making up the eyelids, comprising the application to the eyelids of an eyeshadow according to any one of the preceding claims.

26. Use of an eyeshadow according to any one of Claims 1 to 24, to produce a makeup, deposited on the eyelids, which is homogeneous and/or which has good staying power.

27. Use of xanthan gum and of a mixed silicate, the said mixed silicate being a laponite, and of a glycol containing from 2 to 8 carbon atoms in an eyeshadow containing, in a cosmetically acceptable aqueous medium, a dyestuff in a content of greater than or equal to 10.1% by weight, relative to the total weight of the eyeshadow, to produce a makeup, deposited on the eyelids, which is homogeneous and/or which has good staying power.

## Patentansprüche

1. Lidschatten, der in einem kosmetisch akzeptablen, wässrigen Medium Xanthan, ein gemischtes Silicat, ein Farbmittel, das in einer Menge von mindestens 10,1 Gew.-%, bezogen auf das Gesamtgewicht der Zusammensetzung, enthalten ist, und ein Glycol mit 2 bis 8 Kohlenstoffatomen enthält, wobei es sich bei dem gemischten Silicat um Laponit handelt.

2. Lidschatten nach dem vorhergehenden Anspruch, **dadurch gekennzeichnet, dass** das gemischte Silicat in einer fein verteilten Form vorliegt, wobei die mittlere Größe der Partikel im Bereich von 15 bis 1000 nm liegt.

3. Lidschatten nach einem der vorhergehenden Ansprüche, **dadurch gekennzeichnet, dass** das gemischte Silicat in einer Menge von 0,1 bis 10 Gew.-%, bezogen auf das Gesamtgewicht der Zusammensetzung, vorzugsweise 0,5 bis 5 Gew.-% und besonders bevorzugt 1 bis 4 Gew.-% enthalten ist.

4. Lidschatten nach einem der vorhergehenden Ansprüche, **dadurch gekennzeichnet, dass** das Xanthan in einer Menge von 0,01 bis 10 Gew.-%, bezogen auf das Gesamtgewicht der Zusammensetzung, vorzugsweise 0,1 bis 5 Gew.-% und noch bevorzugter 0,1 bis 3 Gew.-% enthalten ist.

5. Lidschatten nach einem der Ansprüche 1 bis 4, **dadurch gekennzeichnet, dass** das gemischte Silicat und das Xanthan in einer solchen Menge enthalten sind, dass das Gewichtsverhältnis gemischtes Silicat/Xanthan im Bereich von 0,1 bis 20, vorzugsweise 5 bis 15 und noch bevorzugter 8 bis 13 liegt.

6. Lidschatten nach einem der vorhergehenden Ansprüche, **dadurch gekennzeichnet, dass** er mindestens 20 Gew.-% Wasser, bezogen auf das Gesamtgewicht der Zusammensetzung, enthält.

7. Lidschatten nach dem vorhergehenden Anspruch, **dadurch gekennzeichnet, dass** das Wasser in einem Mengenanteil von 20 bis 89,7 Gew.-%, bezogen auf das Gesamtgewicht der Zusammensetzung, vorzugsweise 35 bis 89,7 Gew.-% und noch bevorzugter 40 bis 89,7 Gew.-% enthalten ist.

8. Lidschatten nach einem der vorhergehenden Ansprüche, **dadurch gekennzeichnet, dass** er einen C₂₋₆-Monoalkohol enthält, wie Ethanol, Isopropanol und *n*-Propanol.

9. Lidschatten nach dem vorhergehenden Anspruch, **dadurch gekennzeichnet, dass** der C₂₋₆-Monoalkohol in einer Menge von 0,1 bis 20 Gew.-%, bezogen auf das Gesamtgewicht der Zusammensetzung, und vorzugsweise 0,5 bis 15 Gew.-% enthalten ist.

10. Lidschatten nach einem der vorhergehenden Ansprüche, **dadurch gekennzeichnet, dass** das Farbmittel unter den Pigmenten, Perlglanzpigmenten, Pailletten, wasserlöslichen Farbstoffen und deren Gemischen ausgewählt ist.

11. Lidschatten nach einem der vorhergehenden Ansprüche, **dadurch gekennzeichnet, dass** das Farbmittel unter Titandioxid, Oxiden von Zirconium oder Cer, Oxiden von Zink, Eisen oder Chrom, Manganviolett, Ultramarinblau, Chromhydrat, Eisenblau, Ruß, Lacken auf der Basis von Cochenille-Karmin, Barium, Strontium, Calcium, Aluminium; mit Titan oder Bismutoxidchlorid bedeckten Glimmer-Pigmenten, Titan-Glimmer-Pigmenten mit Eisenoxiden, Titan-Glimmer-Pigmenten mit Eisenblau oder Chromoxid, Perlglanzpigmenten auf der Basis von Bismutoxidchlorid ausgewählt ist.

12. Lidschatten nach einem der vorhergehenden Ansprüche, **dadurch gekennzeichnet, dass** das Farbmittel in einer Menge von insbesondere 10,1 bis 50 Gew.-%, bezogen auf das Gesamtgewicht der Zusammensetzung, vorzugsweise 10,1 bis 40 Gew.-% und noch bevorzugter 15 bis 30 Gew.-% enthalten ist.

13. Lidschatten nach einem der vorhergehenden Ansprüche, **dadurch gekennzeichnet, dass** das Glycol unter Propylenglycol, Ethylenglycol, 1,3-Butylenglycol und Dipropylenglycol ausgewählt ist.

14. Lidschatten nach einem der vorhergehenden Ansprüche, **dadurch gekennzeichnet, dass** das Glycol in einer Menge von 0,1 bis 30 Gew.-%, bezogen auf das Gesamtgewicht der Zusammensetzung, und vorzugsweise 5 bis 20 Gew.-% enthalten ist.

15. Lidschatten nach einem der vorgehenden Ansprüche, **dadurch gekennzeichnet, dass** er als Öl-in-Wasser-Emulsion vorliegt.

16. Lidschatten nach dem vorgehenden Anspruch, **dadurch gekennzeichnet, dass** er eine Fettphase enthält, die mindestens ein Öl aufweist.

17. Lidschatten nach dem vorhergehenden Anspruch, **dadurch gekennzeichnet, dass** das Öl unter den Kohlenwasserstoffölen und den Siliconölen ausgewählt ist.

18. Lidschatten nach Anspruch 16 oder 17, **dadurch gekennzeichnet, dass** das Öl in einer Menge von 0,1 bis 30 Gew.-%, bezogen auf das Gesamtgewicht der Zusammensetzung, vorzugsweise 1 bis 20 Gew.-% und noch bevorzugter 5 bis 15 Gew.-% enthalten ist.

19. Lidschatten nach einem der Ansprüche 16 bis 18, **dadurch gekennzeichnet, dass** die Fettphase eine zusätzliche Fettsubstanz enthält, die unter den Wachsen, Gummis und pastösen Stoffen ausgewählt ist.

20. Lidschatten nach dem vorhergehenden Anspruch, **dadurch gekennzeichnet, dass** die zusätzliche Fettsubstanz in einer Menge von 0,1 bis 25 Gew.-%, bezogen auf das Gesamtgewicht der Zusammensetzung, und vorzugsweise 1 bis 15 Gew.-% enthalten ist.

21. Lidschatten nach einem der Ansprüche 16 bis 19, **dadurch gekennzeichnet, dass** er einen Emulgator enthält.

22. Lidschatten nach dem vorhergehenden Anspruch, **dadurch gekennzeichnet, dass** der Emulgator unter den N-Acylaminosäuren, Aminoxiden, Acylglutamaten, Carbonsäuren und ihren Salzen, Phosphorsäureestern und ihren Salzen, Sulfosuccinaten, Alkylethersulfaten, Sulfosuccinaten, Isethionaten, Alkylimidazolidiniumverbindungen; Ammoniumsalzen; Estern und Ethern von Zuckern, Polyolfettsäureestern, Glycerylethern, ethoxylierten und/oder propoxylierten Fettalkoholethern, Polyethylenglycolfettsäureestern, Copolymeren von Propylenoxid und Ethylenoxid, Dimethiconcopolyolen, Dimethiconcopolyolphosphaten, Dimethiconcopolyolbenzoaten ausgewählt ist.

23. Lidschatten nach Anspruch 21 oder 22, **dadurch gekennzeichnet, dass** der Emulgator in einer Menge von 0,1 bis 10 Gew.-%, bezogen auf das Gesamtgewicht der Zusammensetzung, vorzugsweise 0,5 bis 8 Gew.-% und noch bevorzugter 0,5 bis 5 Gew.-% enthalten ist.

24. Lidschatten nach einem der vorhergehenden Ansprüche, **dadurch gekennzeichnet, dass** er einen kosmetischen Zusatzstoff enthält, der unter den Füllstoffen, Konservierungsmitteln, Parfums, Vitaminen, Hydratisierungsmitteln, beruhigenden Stoffen, Sonnenschutzfiltern, filmbildenden Polymeren, Maskierungsmitteln, Alkalisierungsmitteln oder Ansäuerungsmitteln ausgewählt ist.

25. Verfahren zum Schminken der Augenlider, das das Aufbringen eines Lidschattens nach einem der vorhergehenden Ansprüche auf die Lider umfaßt.

26. Verwendung eines Lidschattens nach einem der Ansprüche 1 bis 24 zur Herstellung einer Schminke, die nach dem Aufbringen auf die Lider homogen ist und/oder eine gute Haftung aufweist.

27. Verwendung von Xanthan, eines gemischten Silicats, wobei es sich bei dem gemischten Silicat um Laponit handelt und eines Glycols mit 2 bis 8 Kohlenstoffatomen in einem Lidschatten, der in einem kosmetisch akzeptablen, wässrigen Medium ein Farbmittel in einer Menge von mindestens 10,1 Gew.-%, bezogen auf das Gesamtgewicht des Lidschattens, enthält, zur Bildung einer Schminke, die nach dem Aufbringen auf die Lider homogen ist und/oder eine hohe Haftung besitzt.
